# EUROPEAN PATENT SPECIFICATION

(11) **EP 3 416 940 B1**
(45) Date of publication and mention of the grant of the patent: **30.10.2019**
(21) Application number: 17707735.1
(22) Date of filing: 15.02.2017
(51) Int. Cl.: C07C 209/48, C07C 211/09, C07C 211/12

(54) **PROMOTER FOR SELECTIVE NITRILE HYDROGENATION**
PROMOTOR FÜR SELEKTIVE NITRILHYDRIERUNG
PROMOTEUR D'HYDROGÉNATION SÉLECTIVE DE NITRILE

(30) Priority: 16.02.2016 US 201662295862 P
(43) Date of publication of application: 26.12.2018
(73) Proprietor: INVISTA Textiles (U.K.) Limited, Manchester M2 3DE (GB)
(72) Inventor: AKI, Sudhir N.V.K., Katy, Texas 77494 (US); RIESTERER, Douglas J., Victoria, Texas 77904 (US)
(74) Representative: Michalski Hüttermann & Partner Patentanwälte mbB
(86) International application number: PCT/US2017/017894
(87) International publication number: WO 2017/142898

(56) References cited:
- WO-A1-98/11059
- WO-A1-03/035250
- DE-A1- 19 809 688

## Description

### FIELD

The disclosures herein relate to a process for hydrogenating a dinitrile comprising contacting the dinitrile with hydrogen in the presence of an heterogeneous iron catalyst. More particularly, the heterogeneous catalyst comprises at least 90 wt.% iron in the presence of promoter comprising at least one selected from alkali metal and alkaline earth metal promoters.

### BACKGROUND

Processes for the hydrogenation of compounds comprising nitrile groups to amine, aminonitrile, or mixtures thereof are known. Hydrogenation of dinitriles to the corresponding diamines is a process which has been used for a long time, in particular the hydrogenation of adiponitrile (ADN) to hexamethylenediamine (HMD), a basic material in the preparation of nylon 6,6. Another industrial process is to hydrogenate a branched dinitrile, such as 2-methylglutaronitrile (MGN), to its corresponding diamine, 2-methylpentamethylenediamine (MPMD).

There has been an increasing interest in recent years in the hydrogenation (also sometimes known as semihydrogenation) of aliphatic dinitriles to aminonitriles, in particular the hydrogenation of adiponitrile to 6-aminocapronitrile (ACN), resulting either directly, or via caprolactam, in nylon-6.

U.S. Patent 4,064,172 discloses an adiponitrile hydrogenation process using iron oxide catalyst where the atomic ratio of oxygen to iron is 1.2:1 to 1.4:1 and the iron catalyst is activated by heating in hydrogen.

U.S. Patent 5,151,543 discloses a process for the selective hydrogenation of aliphatic dinitriles to the corresponding aminonitriles, at 25-150° C. and under a pressure of greater than atmospheric pressure, in the presence of a solvent in a molar excess of at least 2/1 with respect to the dinitrile, the solvent comprising liquid ammonia or an alcohol with 1 to 4 carbon atoms and an inorganic base which is soluble in the said alcohol, in the presence of a Raney catalyst, the aminonitrile obtained being recovered as main product.

U.S. Patents 6,222,059 and 6,278,023 disclose methods for hydrogenating nitriles over an iron catalyst with a promoter selected from aluminum, silicon, zirconium, titanium and vanadium, and a compound based on an alkali metal or an alkaline earth metal.

U.S. Patent 6,255,521 discloses a nitrile hydrogenation method using an iron catalyst with a promoter selected from aluminum, silicon, zirconium, titanium and vanadium, a compound based on an alkali metal or an alkaline earth metal, and phosphorus.

U.S. Patent 6,265,602 discloses a nitrile hydrogenation method using a fixed bed catalyst containing Na, K, Rb, Cs, Mg, Ca, Sr, Ba or Mn, or mixtures thereof, in the form of a basic salt.

U.S. Patent 6,297,394 Iron-based catalyst for hydrogenating alpha-, omega-dinitriles using an iron catalyst with a promoter selected from aluminum, silicon, zirconium, titanium and vanadium, a compound based on an alkali metal or an alkaline earth metal, and manganese.

U.S. Patent 7,115,781 discloses an oxidic composition suitable as a catalyst having divalent and trivalent iron in a specified ratio and oxygen as a counterion. The catalyst is said to be useful for hydrogenation of nitriles to amines.

Published PCT Application WO2015/038673 discloses method for mitigating the rise in pressure drop across a fixed bed process for the hydrogenation of an organonitrile in the presence of a heterogenous iron catalyst.

Further processes are disclosed in the DE 198 09 688, WO 98/11059 and WO 03/035250.

### SUMMARY

Disclosed is a process for hydrogenating a dinitrile comprising contacting the dinitrile with hydrogen over catalyst comprising at least 90 wt.% iron in the presence of promoter comprising at least one selected from alkali metal and alkaline earth metal promoters, wherein:
a. the sum of the concentration of the promoters is from 0.3% to 0.7% of the total weight of catalyst; and
b. the promoter is present depending on the choice of the cation of the promoter, such that at least one of the following apply:
   i. the promoter contains calcium and sodium, more calcium is present than sodium on a molar equivalent basis, and (a) the catalyst comprises at least 200 ppmw sodium and/or (b) the catalyst comprises at least 1000 ppmw calcium;
   ii. the promoter contains calcium and potassium, more calcium is present than potassium on a molar equivalent basis, and (a) the catalyst comprises at least 200 ppmw potassium and/or the catalyst comprises at least 1000 ppmw calcium; and
   iii. the promoter contains calcium and magnesium, and the catalyst comprises at least 1000 ppmw calcium.

In one aspect, the promoter contains calcium and magnesium, and approximately equimolar amounts of calcium and magnesium are present. For example, the ratio of calcium to magnesium may be from 1:1.2 to 1.2:1 on a molar equivalent basis.

In one aspect, the process further comprises decreasing formation of the sum of HMI and BHMT by at least 5 wt.%, based on weight of the sum of HMI and BHMT produced by substantially the same catalyst under substantially the same process conditions except without the promoter.

In another aspect, the process further comprises selecting promoter concentration such that selectivity to AMCPA is increased less than 5 wt.% based on weight of AMCPA produced by substantially the same catalyst under substantially the same process conditions except without the promoter.

In one aspect of the process, the promoter comprises at least two of sodium, calcium and magnesium.

In one aspect of the process, the dinitrile is adiponitrile and further comprises controlling the promoter concentration to reduce selectivity to HMI and BHMT.

In another aspect, the process comprises controlling the promoter concentration below a level that causes the process to produce more AMCPA per unit of adiponitrile feed than operating the process under equivalent conditions except for the absence of the promoter.

Also disclosed is an impurity reduction method for conversion of adiponitrile to hexamethylenediamine comprising contacting adiponitrile with iron catalyst in the presence of a stoichiometric excess of hydrogen, wherein the iron catalyst contains at least enough calcium to decrease selectivity to HMI and BHMT but less than the amount sufficient to increase selectivity to AMCPA, wherein all selectivities are compared to hydrogenation of ADN over substantially the same iron catalyst at substantially the same process conditions in the absence of the calcium.

In one aspect of the process, the concentration of cation promoter is inversely proportional to the dinitrile concentration. For example, if a fixed bed reactor is used, catalyst with different levels of promoter can be loaded into different zones of the fixed bed reactor. In such an embodiment, the catalyst closer to the reactor outlet can have a promoter concentration that is higher than the catalyst closer to the reactor inlet. Similarly, if two or more reactors are used in series, the catalyst in the last reactor in series can have a higher concentration of promoter than the catalyst in the first reactor in the series.

### DETAILED DESCRIPTION

### Detailed Description of the Catalyst

One catalyst suitable for use with the disclosed process can be prepared according to the examples of U.S. Patent 4,064,172.

Another suitable method for preparing catalyst for the disclosed process is taught in International Patent Application Publication No. WO2015/164699A1.

The catalyst in the disclosed process is a hydrogenation catalyst suitable for hydrogenating a dinitrile to a diamine or a mixture of diamine and aminonitrile. Such catalysts may comprise Group VIII elements including iron, cobalt, nickel and combinations thereof. For example, the catalyst can comprise 90 to 99.9 wt.% iron, for example, 95 to 99.5 wt.% iron, for example, 95 to 99 wt.% iron.

In one embodiment, the hydrogenation catalyst may comprise of iron oxide, such as obtained as magnetite ore, which may be further processed to yield the Fe²⁺ / Fe³⁺ ratio in the catalyst to be in the 0.65 to 0.75 range. In other embodiments, the iron may be from other commercially available ores.

The catalyst can comprise metallic iron, for example, up to 99.99 wt.% metallic iron. Alternatively, the catalyst can be a mixture of metallic iron, iron oxides, iron sulfides and other iron-containing compounds. The catalyst can comprise direct-reduced iron, also referred to as sponge iron.

In some embodiments, the catalyst contains from 90% to 99.99% of metallic iron. In other embodiments, the catalyst contains from 95% to 99.9% of metallic iron. In some other embodiments, the catalyst contains from 98% to 99.8% of metallic iron.

Catalysts comprising Group VIII metals are described in U.S. Patent No. 6,376,714.

The catalyst can be present in any appropriate physical shape or form. It can be in fluidizable forms, extrudates, tablets, spheres, or combinations of two or more thereof. When employing the process using a fixed bed catalyst, the catalyst is in the form of granules having a particle size in the range of about 0.03 to 0.40 inch (0.76 to 10.2 mm). When employing the process using a slurry-phase catalyst, the catalyst is in finely divided form, preferably less than about 100 µ in size, most preferred range being 20 to 75µ. The catalyst may be supported or unsupported.

In a preferred mode of operation the process is conducted on a continuous basis in a continuous stirred tank reactor (CSTR) or a slurry bubble column reactor (SBCR) or a plug flow reactor (PFR), or a trickle bed reactor. An example of a bubble column reactor, which is not confined to this reaction, has been described in U.S. Pat. No 4,429,159. Descriptions of plug flow and continuous stirred tank reactors have been delineated in the book entitled, "Chemical Reaction Engineering" written by Octave Levenspiel. The preference for reactor is not meant to limit the disclosure, which can also be conducted in batch or semi-batch mode.

A catalyst may be prepared by reducing an oxide of a Group VIII metal with hydrogen. For example, a catalyst may activated by reducing at least a part of iron oxide to metallic iron by heating it in the presence of hydrogen at a temperature above 200° C but not above 600° C. Activation may be continued until at least 80% by weight of the available oxygen in the iron has been removed and may be continued until substantially all, for example from 95 to 98% of the available oxygen has been removed. During the activation it is desirable to prevent back-diffusion of water-vapor formed. Examples of catalyst activation techniques are described in U.S. Patent No. 3,986,985.

### Detailed Description of the Promoter

The alkali metal and alkaline earth metal promoter can be added to the catalyst by any suitable method. For example, a catalyst precursor can be contacted with an aqueous solution of calcium hydroxide and then dried in ambient or heated air. Other non-limiting examples of aqueous solutions useful for adding promoter to the catalyst or catalyst precursor include calcium carbonate, magnesium hydroxide, magnesium carbonate, potassium hydroxide, potassium carbonate, sodium hydroxide and sodium carbonate.

The liquid contacting method for transferring the alkali metal and alkaline earth metal promoter to the catalyst or catalyst precursor can include incipient wetness impregnation, spraying, rinsing and immersing, merely to name a few contacting methods.

The catalyst and promoter can be separately introduced into contact with dinitrile; however, the catalyst may be precontacted with the promoter. This may be done in water and/or a solvent such as an alcohol, ether, ester, ammonia, or combinations of two or more thereof.

In some embodiments, the addition of promoters may be performed during the fusion process. Such fusion processes are commonly practiced in the catalyst manufacturing industry, which comprise the steps of obtaining a metal ore, melting the ore by any thermal means [electric arc, furnace, etc.], adding promoter types in the desired levels to the molten liquid, allowing the melt to equilibrate and then fusing the melt back into the solid for preparing the catalyst. The prepared catalyst may be in the form of granules, powders with the desired particle size distribution, pelletized solids, or any other suitable form.

Preferably, the precontacting is also carried out in the presence of hydrogen. Contacting of the catalyst and promoter produces a pretreated catalyst. The pretreated catalyst can be washed with a solvent disclosed above, preferably under anaerobic conditions to produce a promoter-treated catalyst.

The contacting of the catalyst and promoter can be carried out under any conditions effective to produce a promoter-treated catalyst that can improve selective hydrogenation of a dinitrile to a diamine and/or aminonitrile, e.g. adiponitrile to hexamethylenediamine and/or 6-aminocapronitrile. Generally, the entire process for producing the promoter-treated catalyst can be carried out by contacting a catalyst with a promoter disclosed above at a temperature in the range of from about 20° C. to about 150° C., preferably about 30° C. to about 100° C., under the same general pressures as described earlier, for about 5 seconds to about 25 hours.

In some embodiments, the sum of the concentration of the promoters is from 0.3% to 0.7% of the total weight of catalyst.

In some embodiments, the promoter is present in molar ratios depending on the choice of cation, such that if the promoter contains calcium and sodium, then more calcium is present than sodium.

In some embodiments, the promoter is present in molar ratios depending on the choice of cation, such that if the promoter contains calcium and potassium, then more calcium is present than potassium.

In some embodiments, the promoter is present in molar ratios depending on the choice of cation, such that if the promoter is calcium and magnesium, then approximately equimolar amounts are present.

In some embodiments, the promoter contains calcium and magnesium, and approximately equimolar amounts of calcium and magnesium are present. For example, the ratio of calcium to magnesium may be from 1:1.2 to 1.2:1 on a molar equivalent basis.

In some embodiments, the promoter comprises at least two of sodium (Na), calcium (Ca) and magnesium (Mg). In other embodiments, the promoter comprises potassium (K).

In some embodiments, the molar ratio of Ca:Na in the catalyst is at least 5:2. In other embodiments, the molar ratio of Ca:Na in the catalyst is at least 7:2. In some embodiments, the molar ratio of Ca:Na in the catalyst is at least 5:1.

In some embodiments, the molar ratio of Ca:K in the catalyst is at least 5:2. In other embodiments, the molar ratio of Ca:K in the catalyst is at least 7:2. In some embodiments, the molar ratio of Ca:K in the catalyst is at least 5:1.

In some embodiments, the promoter is present in the reactor at concentration from 500 ppmw to 3500 ppmw based on weight of the catalyst. In other embodiments, the promoter is present in the reactor at concentration from 1000 ppmw to 3000 ppmw based on weight of the catalyst. In some other embodiments, the promoter is present in the reactor at concentration from 1500 ppmw to 3000 ppmw based on weight of the catalyst.

In some embodiments, the catalyst comprises 200 ppmw to 800 ppmw sodium, based on weight of the catalyst. In other embodiments, the catalyst comprises 300 to 700 ppmw sodium, based on weight of the catalyst. In some other embodiments, the catalyst comprises 400 to 600 ppmw sodium, based on weight of the catalyst. In some embodiments, the catalyst contains less than 400 ppmw sodium based on weight of the catalyst.

In some embodiments, the catalyst comprises 100 to 1000 ppmw potassium based on weight of the catalyst. In other embodiments, the catalyst comprises 200 to 800 ppmw potassium based on weight of the catalyst. In other embodiments, the catalyst comprises 200 to 400 ppmw potassium based on weight of the catalyst. In some other embodiments, the catalyst contains less than 400 ppmw potassium based on weight of the catalyst.

In some embodiments, the catalyst comprises less than 4000 ppmw aluminum based on weight of the catalyst. In other embodiments, the catalyst comprises less than 3000 ppmw aluminum based on weight of the catalyst.

Hydrogen can be delivered to the reaction mixture as gas, preferably pure hydrogen. The hydrogen should be delivered at a rate that will maintain hydrogen in molar excess relative to the dinitrile.

Diamine and/or aminonitrile, e.g. hexamethylenediamine and/or 6-aminocapronitrile, can be recovered from the reaction products by typical purification procedures such as recrystallization or preferably, distillation. The unreacted dinitrile can be sent back to the hydrogenation reactor to obtain additional diamine and/or aminonitrile.

Examples of suitable dinitriles may include C₃-C₁₆ aliphatic, cyclic, aromatic, branched, saturated as well as unsaturated dinitriles. Some non-limiting examples are adiponitrile, methylglutaronitrile, succinonitrile, glutaronitrile, ethylsuccinonitrile, etc. The preferred dinitrile is adiponitrile (ADN) or methylglutaronitrile (MGN).

In some embodiments of the process, the concentration of cation promoter is inversely proportional to the dinitrile concentration.

In some embodiments, the dinitrile concentration decreases from the inlet to the outlet of reaction vessel that houses the cation promoter catalyst. In other embodiments, a series of vessels may house the cation promoter catalyst. Examples may include, but not limited to, a series of batch or continuous stirred-tank reactors with flowline connections, fixed-bed reactors, plug-flow reactors, fluidized-bed reactors in series, or combinations thereof. It is understood that the dinitrile concentration decreases as the reaction progresses in the flow direction. The rate of dinitrile concentration decrease may depend on the rate of reaction, amount of dilution, and other operational variations.

The present disclosure is also suitable in the field of partial hydrogenation, such as in the conversion of adiponitrile (ADN) to its partially hydrogenated product 6-aminocapronitrile (ACN). The formation of undesired side products may be reduced substantially when the levels of calcium, magnesium, potassium and sodium as promoters are increased in the reaction system, according to the embodiments of this disclosure.

In some embodiments of the present disclosure, a partially hydrogenated product of a dinitrile such as aminonitrile may be further hydrogenated to a fully hydrogenated diamine product. For example, a feed containing aliphatic dinitrile, such as ADN, may undergo partial hydrogenation to yield 6-aminocapronitrile (ACN), which may be fully hydrogenated to HMD.

The hydrogenated or partially hydrogenated amine product, obtained according to the present disclosure, may be further processed using conventional separation techniques, such as any of the distillative unit operation, membrane separation, film evaporation, short-path distillation (SPD), extraction, precipitation, filtration or combinations thereof. Such chemical separation techniques are commonly used to recover products from light- and/or high-boiling impurities, and are well-known to a skilled persion in chemical processing industry. The desired product according to this disclosure may be recovered in high yield and purity due to the impurity reduction as against that obtained in a system where the calcium, magnesium, potassium and sodium promoter levels are not increased and/or their mole ratios are not maintained according to the embodiments of this disclosure.

### Definitions

ADN means "adiponitrile".

HMD means "hexamethylenediamine".

ACN means "6-aminocapronitrile".

HMI means "hexamethyleneimine".

DCH means "1,2-diaminocylohexane".

BHMT means "bis(hexamethylene)triamine".

AMCPA means "aminomethyl cyclopentylamine".

MGN means "2-methylglutaronitrile".

MPMD means "2-methylpentamethylenediamine".

3-MPIP means "3-methylpiperidine".

MCPD means "methylcyclopentanediamine".

CaO means calcium oxide, MgO means magnesium oxide, K₂O means potassium oxide, and Na₂O means sodium oxide.

The term "ppm" or "ppmw" means parts per million by weight unless otherwise stated.

The term "dinitrile" means an organic compound comprising two nitrile (-C≡N) groups, for example ADN.

The term "diamine" means an organic compound comprising two amine (-NH₂) groups, for example HMD.

Psig is pounds per square inch gauge pressure.

### EXAMPLES

### Test Equipment used in the Examples

A 1-liter, batch autoclave reactor (Manuf.: Autoclave Engineer), equipped with an agitator, electric heating jacket and internal cooling coils, a MaxPro hydrogen compression system, a 2-liter hydrogen supply reservoir and associated flow lines, control valves and process instrumentation, is used. The autoclave body and seal ring are made of 316 SS. The reactor is capable of handling pressures of 413,7 bar (6000 psig) and temperatures in excess of 648 °C (1200 °F). Agitation is accomplished by Series 0.75 MagneDrive II stirrer capable of speeds up to 1200 RPM. The reactor contents are charged using a threaded feed port on the top face of the body. The reaction effluent is recovered from the bottom drain valve.

### Feeds used in the Examples

The autoclave reactor feeds consist of dry hydrogen, ammonia, reactants and pre-activated metallic iron catalyst. All except hydrogen are batch-fed at the start of each run. Only hydrogen is continuously replenished to the unit at a controlled rate that more or less matches with its consumption during the reaction.

Dry hydrogen gas is fed from its supply cylinder to a MaxPro Maximator compressor capable of compressing hydrogen up to 689, 5 bar (10,000 Psig) discharge pressure. The compressed hydrogen gas is stored in a 2-liter self-sealing stainless steel reservoir. The reservoir provides continuous supply of pressurized hydrogen to the reactor during testing. A pressure regulator device, installed between the hydrogen reservoir and autoclave reactor, provides the precious control of the reactor pressure by supplying as-needed hydrogen gas to the reaction.

Compressed hydrogen is continuously fed from the reservoir to the autoclave during the reaction. The regulator controls the necessary flow to the unit as hydrogen is consumed in the reaction. The reservoir pressure depletes at a rate equal to the hydrogen uptake in hydrogenation reaction. The reservoir pressure versus time is continuously recorded throughout the reaction and is used to calculate hydrogen consumption in the reaction. The reaction progress is monitored from the pressure-time data.

Anhydrous ammonia is supplied out of a cylinder through a liquid discharge tube. The liquid ammonia is charged to a 2-liter SS vessel that is mounted on a calibrated weighing scale. The amount of ammonia charged to the system is monitored using the scale reading.

Appropriate amounts of organic dinitrile and activated metallic iron catalyst are batch-fed to the reactor for each test.

### Catalyst Preparation, Activation and Recovery

Coarse catalyst pellets supplied by commercial catalyst vendors are ground using a tungsten carbide vial and two tungsten carbide balls. The vial is tightened in a holder inside a SPEX/Mixer/Mill. The contents are shaken for four minutes and sieved to yield between 25 and 60 mesh size distribution. The fines from the grinding process are discarded. Catalyst size reduction is desirable to be able to recover the catalyst-containing reactor effluent from the autoclave bottom drain valve.

The above ground catalyst is activated in a 1-inch x 16-inch long catalyst activation tube. The 316-SS activation tube is secured in an electric, clamshell furnace.

Activation of the ground iron oxide catalyst is performed by reducing iron oxide to metallic iron (Fe⁰) under the hydrogen environment. Sufficient quantity of iron oxide catalyst is activated to metallic iron by heating in the presence of hydrogen at a temperature above 200°C but not above 600°C. Activation is continued until at least 80% by weight of the molecular oxygen in the iron has been removed and may be continued until substantially all, for example from 95 to 98% of the available oxygen has been removed. During the activation it is desirable to prevent back-diffusion of water vapor formed as a result of the reduction process.

Once the activation is complete, the metallic iron catalyst is often pyrophoric and is protected from atmospheric oxygen exposure. The activated metallic iron catalyst is recovered from the activation step and blanketed with an inert gas, such as nitrogen, and maintained in the inert atmosphere until further use.

Typically, about 85g of the starting ground iron oxide catalyst charge yields between 60-65g of activated metallic iron catalyst.

### Example 1: Hydrogenation of dinitrile (reference example)

ADN and activated metallic iron catalyst, prepared according to the method described above, are premixed in an oxygen-deficient environment, such as a dry glove box, and batch-fed to the autoclave reactor described above. The reactor is sealed and purged with 13,8 bar (200-Psig) nitrogen several times to remove any trapped air from the system. Anhydrous ammonia is next metered into the reactor using the weighing scale. Agitation is started at about 1200 RPM and the contents temperature is gradually increased to the set point of about 150 °C and controlled there.

A continuous stream of hydrogen gas is next admitted to the reactor from the reservoir wherein the reactor operating pressure is controlled by controlling the hydrogen feed rate. The reservoir pressure versus time is monitored and recorded. The bulk temperature in the reactor is controlled to within ±2 °C by the internal cooling coil.

The hydrogenation reaction is considered complete when the reservoir pressure ceases to fall with time. The reactor is then cooled to near-ambient conditions and de-pressurized. Nitrogen purging removes any residual hydrogen and ammonia trapped in the reactor head space. The feed plug is unscrewed and the reactor effluent is drained into a clean sample container.

The effluent is GC analyzed for ADN, HMD, ACN, HMI, DCH, BHMT, AMCPA, MGN, 3-MPIP, MPMD and few other organic components.

The reactor effluent is analyzed to contain (by wt.) 0.01% ADN, 0.02% ACN, 0.49% HMI, 0.5% DCH, 1.07% BHMT, 0.11% AMCPA, and balance HMD which is the desired hydrogenation product. The concentrations of calcium (present as CaO), magnesium (present as MgO), potassium (present as K₂O) and sodium (present as Na₂O) in the supplied commercial iron oxide "catalyst A" are about 200 ppm, about 3000 ppm, about 50 ppm and about 15 ppm, respectively. The iron oxide "Catalyst A" is activated to metallic iron using the catalyst activation procedures described above.

### Examples 2-4: Hydrogenation of dinitrile with increased calcium promoter

The procedure described in Example 1 is repeated using the same equipment and feeds described above except the concentration of calcium (present as CaO) in the reaction system is increased.

The Table below is a summary of the,measured composition of the reactor effluent for each case. The balance is HMD which is the desired hydrogenation product. As an example, the HMD content in the reactor effluent is 97.8 wt% in the case of Example 1.

| **Ex.** | **System** | **Ca ppm** | **Na ppm** | **ADN wt%** | **ACN wt%** | **HMI wt%** | **DCH wt%** | **BHMT wt%** | **AMCPA wt%** |
|---|---|---|---|---|---|---|---|---|---|
| 1 | Catalyst A | 200 | 15 | 0.01 | 0.02 | 0.49 | 0.50 | 1.07 | 0.11 |
| 2 | + Calcium | 1000 | 15 | 0.21 | 0.02 | 0.33 | 0.55 | 0.98 | 0.05 |
| 3 | " | 2000 | 15 | 0.02 | 0.04 | 0.17 | 0.54 | 0.65 | 0.07 |
| 4 | " | 5000 | 15 | 0.03 | 0.06 | 0.17 | 0.56 | 0.68 | 0.29 |

### Example 5: Hydrogenation of dinitrile with increased sodium promoter (reference example)

The procedure described in Example 1 is repeated using the same equipment and feeds described above except the concentration of sodium (present as Na₂O) in the reaction system is increased.

The Table below is a summary of the measured composition of the reactor effluents. The balance is HMD which is the desired hydrogenation product.

| **Ex.** | **System** | **Ca ppm** | **Na ppm** | **ADN wt%** | **ACN wt%** | **HMI wt%** | **DCH wt%** | **BHMT wt%** | **AMCPA wt%** |
|---|---|---|---|---|---|---|---|---|---|
| 1 | Catalyst A | 200 | 15 | 0.01 | 0.02 | 0.49 | 0.50 | 1.07 | 0.11 |
| 5 | + Sodium | 200 | 400 | 0.07 | 0.10 | 0.35 | 0.53 | 0.96 | 0.04 |

### Example 6: Hydrogenation of dinitrile with increased calcium and sodium promoters

The procedure described in Example 1 is repeated using the same equipment and feeds described above except the concentrations of calcium (present as CaO) and sodium (present as Na₂O) in the reaction system are increased.

The Table below is a summary of the measured composition of the reactor effluents. The balance is HMD which is the desired hydrogenation product.

| **Ex.** | **System** | **Ca ppm** | **Na ppm** | **ADN wt%** | **ACN wt%** | **HMI wt%** | **DCH wt%** | **BHMT wt%** | **AMCPA wt%** |
|---|---|---|---|---|---|---|---|---|---|
| 1 | Catalyst A | 200 | 15 | 0.01 | 0.02 | 0.49 | 0.50 | 1.07 | 0.11 |
| 6 | + Calcium + Sodium | 2000 | 400 | 0.05 | 0.06 | 0.13 | 0.50 | 0.49 | 0.15 |

The unexpected synergistic effect of increasing both, calcium and sodium levels, along with their mole ratios in the reaction system is evidenced by HMI and BHMT reductions without any substantial increase in AMCPA formation as shown below.

| **Ex.** | **Ca ppm** | **Na ppm** | **ADN wt%** | **ACN wt%** | **HMI wt%** | **DCH wt%** | **BHMT wt%** | **AMCPA wt%** |
|---|---|---|---|---|---|---|---|---|
| 1 | 200 | 15 | 0.01 | 0.02 | 0.49 | 0.50 | 1.07 | 0.11 |
| 5 | 200 | 400 | 0.07 | 0.10 | 0.35 | 0.53 | 0.96 | 0.04 |
| 3 | 2000 | 15 | 0.02 | 0.04 | 0.17 | 0.54 | 0.65 | 0.07 |
| 6 | 2000 | 400 | 0.05 | 0.06 | 0.13 | 0.50 | 0.49 | 0.15 |

### Example 7: Hydrogenation of dinitrile with increased calcium and sodium promoters

The procedure described in Example 1 is repeated using the same equipment and feeds described above except using iron oxide "catalyst B" from another source. The concentrations of calcium (present as CaO), magnesium (present as MgO), potassium (present as K₂O) and sodium (present as Na₂O) in the iron oxide "catalyst B" are about 1300 ppm, about 3000 ppm, about 200 ppm and about 90 ppm, respectively. The iron oxide "Catalyst B" is activated to metallic iron using the catalyst activation procedures described above.

The table below is a summary of the measured composition of the reactor effluents in Examples 1 and 7. The balance is HMD which is the desired hydrogenation product.

| **Ex.** | **System** | **Ca ppm** | **Mg ppm** | **Na ppm** | **ADN wt%** | **ACN wt%** | **HMI wt%** | **DCH wt%** | **BHMT wt%** | **AMCPA wt%** |
|---|---|---|---|---|---|---|---|---|---|---|
| 1 | Catalyst A | 200 | 3000 | 15 | 0.01 | 0.02 | 0.49 | 0.50 | 1.07 | 0.1 1 |
| 7 | Catalyst B | 1300 | 3000 | 90 | 0 | 0 | 0.26 | 0.51 | 0.75 | 0.12 |

### Example 8: Hydrogenation of dinitrile with increased calcium, magnesium and sodium promoters

The procedure described in Example 1 is repeated using the same equipment and feeds described above except using iron oxide "catalyst C" from another source. The concentrations of calcium (present as CaO), magnesium (present as MgO), potassium (present as K₂O) and sodium (present as Na₂O) in the iron oxide "catalyst C" are about 2000 ppm, 3500 ppm, 200 ppm and about 90 ppm, respectively. The iron oxide "Catalyst C" is activated to metallic iron using the catalyst activation procedures described above.

The table below is a summary of the measured composition of the reactor effluents in Examples 1, 7 and 8. The balance is HMD which is the desired hydrogenation product.

| **Ex.** | **System** | **Ca ppm** | **Mg ppm** | **Na ppm** | **ADN wt%** | **ACN wt%** | **HMI wt%** | **DCH wt%** | **BHMT wt%** | **AMCPA wt%** |
|---|---|---|---|---|---|---|---|---|---|---|
| 1 | Catalyst A | 200 | 3000 | 15 | 0.01 | 0.02 | 0.49 | 0.50 | 1.07 | 0.11 |
| 7 | Catalyst B | 1300 | 3000 | 90 | 0 | 0 | 0.26 | 0.51 | 0.75 | 0.12 |
| 8 | Catalyst C | 2000 | 3500 | 90 | 0.01 | 0.02 | 0.09 | 0.54 | 0.29 | 0.55 |

### Examples 9a-b: Hydrogenation of branched dinitrile with increased calcium, magnesium and sodium promoters

Hydrogenation of MGN to MPMD is carried out in the presence of hydrogen and over each activated metallic iron catalyst (source: iron oxide "catalyst A" and "catalyst B"). The procedures and reaction conditions described above, the 1-liter batch autoclave setup and H₂/NH₃ feeds described above are employed in this example. It is observed that the formation of undesired side products, mainly 3-MPIP and high boilers, is reduced substantially when the levels of calcium, magnesium and sodium as promoters are increased in the reaction system according to the embodiments of this disclosure. The conversion of MGN to MPMD is complete.

The table below is a summary of the measured composition of the reactor effluents in the present example. The balance is MPMD which is the desired MGN hydrogenation product.

| **Ex.** | **System** | **Ca ppm** | **Mg ppm** | **Na ppm** | **3-MPIP wt%** | **MCPD wt%** | **High boilers wt%** |
|---|---|---|---|---|---|---|---|
| 9a | Catalyst A | 200 | 3000 | 15 | 4.51 | 1.03 | 1.33 |
| 9b | Catalyst C | 2000 | 3500 | 90 | 3.3 | 1.05 | 0.44 |

The unexpected synergistic effect of increasing calcium, magnesium and sodium levels in the MGN hydrogenation reaction system is evidenced by 3-MPIP byproduct and high boilers reductions in Examples 9a-b.

### Example 10: Hydrogenation of dinitrile with increased promoter levels

A continuous, fixed-bed hydrogenation facility converts ADN to HMD in the presence of hydrogen, ammonia and activated metallic iron catalyst (source: iron oxide "catalyst A"). When the promoter levels are increased and their relative mole ratios maintained according to the embodiments of this disclosure, an appreciable and desired reduction in the formation of impurities, mainly HMI and BHMT, is observed with corresponding increase in the HMD product yield. There is no AMCPA increase observed.

### Example 11: Hydrogenation of dinitrile using series converters and increased promoter levels

A series of hydrogenation converters is operated to convert the ADN feed to HMD in the presence of hydrogen, ammonia and activated metallic iron "catalyst A" with the promoter levels described in Example 2. Fresh ADN-rich feed enters the first converter and the partially hydrogenated effluent from the first converter becomes feed to the second converter. The partially hydrogenated effluent from the second converter further becomes feed to the third converter and so on. An atleast two converters in series may be employed, although there is no limit as to the number of converters used in series arrangement.

The converters may be operated in batch, semi-batch or continuous mode. Each converter may be designed for a suitable reaction activity, i.e., conversion, selectivity and thermal effects between the inlet and outlet flow streams. Direct or indirect, inter-stage heat exchange with or without bypass flows may be employed for effective temperature management. Other hydrogenation feeds may either be introduced in the first converter or distributed across the various stages depending on the operation and controls.

When the promoter levels in the catalyst are increased versus the Example 1 baseline levels, an appreciable and desired reduction in the formation of impurities, mainly HMI and BHMT, is observed with corresponding increase in the final HMD product yield. The byproduct AMCPA level increases slightly versus that for the Example 1 baseline.

### Example 12: Hydrogenation of dinitrile using series converters and increased promoter levels

The series converter arrangement as described in Example 11 is utilized in this example, except that the promoter level in the activated metal iron catalyst is gradually increased from the first converter to each subsequent converter in series. The ADN-rich feed continues to be hydrogenated as it passes through.

When the promoter levels are gradually increased, an appreciable and desired reduction in the formation of impurities, mainly HMI, BHMT, is observed with corresponding increase in the final HMD product yield. The byproduct AMCPA level shows a similar trend as that observed in Example 11.

### Example 13: Hydrogenation of dinitrile using single vessel and increased promoter levels

A single vessel employs the activated metal iron catalyst, such as any of the "Catalyst A" or "Catalyst B" or "Catalysl C", having increasing promoter levels in the direction of the dinitrile feed flow. The ADN-rich feed continues to be hydrogenated as it passes through the catalyst zone. Reduction in the formation of impurities, mainly HMI, and BHMT, is observed with corresponding increase in the final HMD product yield versus when the baseline catalyst of Example 1 is used.

### Example 14: Hydrogenation of branched dinitrile using single vessel and increased promoter levels

The Example 13 configuration is used for hydrogenating MGN-rich feed into the desired MPMD product. Reduction in the formation of impurities, mainly 3-MPIP and high boilers, is observed with corresponding increase in the final MPMD product yield versus when the baseline catalyst of Example 1 is used.

## Claims

1. A process for hydrogenating a dinitrile comprising contacting the dinitrile with hydrogen over catalyst comprising at least 90 wt.% iron in the presence of promoter comprising at least one selected from alkali metal and alkaline earth metal promoters, wherein:
a. the sum of the concentration of the promoters is from 0.3% to 0.7% of the total weight of catalyst; and
b. the promoter is present depending on the choice of the cation promoter, such that at least one of the following apply:
i. the promoter contains calcium and sodium, more calcium is present than sodium on a molar equivalent basis, and (a) the catalyst comprises at least 200 ppmw sodium and/or (b) the catalyst comprises at least 1000 ppmw calcium;
ii. the promoter contains calcium and potassium, more calcium is present than potassium on a molar equivalent basis, and (a) the catalyst comprises at least 200 ppmw potasium and/or (b) the catalyst comprises at least 1000 ppmw calcium; and
iii. the promoter contains calcium and magnesium, and the catalyst comprises at least 1000 ppmw calcium.

2. The process of claim 1 wherein the dinitrile is adiponitrile and wherein the process further comprises controlling the promoter concentration to reduce selectivity to HMI (hexamethyleneimine) and BHMT (bis(hexamethylene)triamine).

3. The process of claim 2 further comprising controlling the promoter concentration below a level that causes the process to produce more AMCPA (aminomethyl cyclopentylamine) per unit of adiponitrile feed than operating the process under equivalent conditions except for the absence of the promoter.

4. The process of any one of the preceding claims wherein the promoter comprises at least two of sodium, calcium and magnesium.

5. The process of claim 4 wherein the molar ratio of Ca:Na in the catalyst is at least 5:2.

6. The process of any of claims 1, 2 or 3 wherein the promoter contains calcium and potassium and wherein the molar ratio of Ca:K is at least 5:2.

7. The process of any one of the preceding claims wherein the promoter is at least one compound selected from the group consisting of oxides, hydroxides and carbonates.

8. The process of any one of the preceding claims wherein the promoter is added to the catalyst before contacting the catalyst with dinitrile and hydrogen.

9. The process of any one of the preceding claims where the dinitrile is contacted with hydrogen in a reactor and wherein the promoter is present in the reactor at concentration from 500 ppmw to 3500 ppmw based on weight of the catalyst.

10. The process of any one of the preceding claims wherein the promoter comprises Ca(OH)₂.

11. The process of any one of the preceding claims wherein the promoter comprises NaOH.

12. The process of any one of the preceding claims wherein the catalyst comprises 200 ppmw to 800 ppmw sodium.

13. The process of any one of the preceding claims wherein the catalyst comprises 100 to 500 ppmw potassium based on weight of the catalyst.

14. The process of any one of the preceding claims wherein the dinitrile is adiponitrile and wherein the process further comprises decreasing formation of the sum of HMI and BHMT by at least 5 wt.%, based on weight of the sum of HMI and BHMT produced by substantially the same catalyst under substantially the same process conditions except without the promoter.

15. The process of any one of the preceding claims wherein the dinitrile is adiponitrile and wherein the process further comprises selecting promoter concentration such that selectivity to AMCPA is increased less than 5 wt.% based on weight of AMCPA produced by substantially the same catalyst under substantially the same process conditions except without the promoter.

## Patentansprüche

1. Verfahren zum Hydrieren eines Dinitrils, mit dem Inkontaktbringen des Dinitrils mit Wasserstoff über einen Katalysator, der mindestens 90 Gew.-% Eisen enthält, in Gegenwart eines Promotors, der mindestens eine Komponente aufweist, die ausgewählt ist aus Alkalimetall- und Erdalkalimetallpromotoren, wobei:
a. die Summe der Konzentration der Promotoren 0,3% bis 0,7% des Gesamtgewichts des Katalysators beträgt; und
b. der Promotor in Abhängigkeit von der Wahl des Kationenpromotors vorhanden ist, so dass mindestens einer der folgenden Punkte zutrifft:
i: der Promotor enthält Kalzium und Natrium, wobei mehr Kalzium als Natrium auf einer molaren Äquivalentbasis vorhanden ist, und (a) der Katalysator mindestens 200 ppmw Natrium enthält und/oder (b) der Katalysator mindestens 1000 ppmw Kalzium enthält;
ii. der Promotor enthält Kalzium und Kalium, wobei mehr Kalzium als Kalium auf einer molaren Äquivalentbasis vorhanden ist, und (a) der Katalysator mindestens 200 ppmw Kalium enthält und/oder (b) der Katalysator mindestens 1000 ppmw Kalzium enthält; und
iii. der Promotor enthält Kalzium und Magnesium, wobei der Katalysator mindestens 1000 ppmw Kalzium enthält.

2. Verfahren nach Anspruch 1, wobei das Dinitril Adiponitril ist, und wobei das Verfahren ferner das Steuern der Promotorkonzentration zum Reduzieren der Selektivität bezüglich HMI (Hexamethylenimin) und BHMT (Bis(hexamethylen)triamin) aufweist.

3. Verfahren nach Anspruch 2, ferner mit dem Steuern der Promotorkonzentration unter ein Niveau, das bewirkt, dass das Verfahren mehr AMCPA (Aminomethylcyclopentylamin) pro Einheit Adipodinitril-Zufuhr erzeugt als wenn das Verfahren unter äquivalenten Bedingungen mit Ausnahme der Abwesenheit des Promotors ausgeführt wird.

4. Verfahren nach einem der vorhergehenden Ansprüche, wobei der Promotor mindestens zwei Elemente unter Natrium, Kalzium und Magnesium aufweist.

5. Verfahren nach Anspruch 4, wobei das Molverhältnis von Ca:Na im Katalysator mindestens 5:2 beträgt.

6. Verfahren nach Anspruch 1, 2 oder 3, wobei der Promotor Kalzium und Kalium enthält, und wobei das Molverhältnis von Ca:K mindestens 5:2 beträgt.

7. Verfahren nach einem der vorhergehenden Ansprüche, wobei der Promotor mindestens eine Verbindung ist, die aus der Gruppe bestehend aus Oxiden, Hydroxiden und Carbonaten ausgewählt ist.

8. Verfahren nach einem der vorhergehenden Ansprüche, wobei der Promotor dem Katalysator zugesetzt wird, bevor der Katalysator mit Dinitril und Wasserstoff in Kontakt gebracht wird.

9. Verfahren nach einem der vorhergehenden Ansprüche, wobei das Dinitril in einem Reaktor mit Wasserstoff in Kontakt gebracht wird, und wobei der Promotor im Reaktor in einer Konzentration von 500 ppmw bis 3500 ppmw, bezogen auf das Gewicht des Katalysators, vorhanden ist.

10. Verfahren nach einem der vorhergehenden Ansprüche, wobei der Promotor Ca(OH)₂ enthält.

11. Verfahren nach einem der vorhergehenden Ansprüche, wobei der Promotor NaOH enthält.

12. Verfahren nach einem der vorhergehenden Ansprüche, wobei der Katalysator 200 ppmw bis 800 ppmw Natrium enthält.

13. Verfahren nach einem der vorhergehenden Ansprüche, wobei der Katalysator 100 bis 500 ppmw Kalium, bezogen auf das Gewicht des Katalysators, enthält.

14. Verfahren nach einem der vorhergehenden Ansprüche, wobei das Dinitril Adiponitril ist, und wobei das Verfahren ferner das Vermindern der Ausbildung der Summe von HMI und BHMT um mindestens 5 Gew.-%, bezogen auf das Gewicht der Summe von HMI und BHMT, aufweist, die durch im Wesentlichen den gleichen Katalysator unter im Wesentlichen den gleichen Verfahrensbedingungen außer ohne den Promotor hergestellt werden.

15. Verfahren nach einem der vorhergehenden Ansprüche, wobei das Dinitril Adiponitril ist, und wobei das Verfahren ferner das Auswählen der Promotorkonzentration derart aufweist, dass die Selektivität bezüglich AMCPA, bezogen auf das Gewicht von AMCPA, das durch im Wesentlichen den gleichen Katalysator unter im Wesentlichen den gleichen Verfahrensbedingungen außer ohne den Promotor hergestellt wird, um weniger als 5 Gew.-% erhöht ist.

## Revendications

1. Procédé d'hydrogénation d'un dinitrile comprenant la mise en contact du dinitrile avec de l'hydrogène sur un catalyseur comprenant au moins 90 % en poids de fer en présence d'un promoteur comprenant au moins l'un sélectionné parmi les promoteurs métaux alcalins et métaux alcalino-terreux, dans lequel :
a. la somme de la concentration de promoteurs est comprise entre 0,3% et 0,7% du poids total du catalyseur ; et
b. le promoteur est présent suivant le choix du promoteur cationique, de telle sorte qu'au moins une des conditions suivantes s'applique :
i. le promoteur contient du calcium et du sodium, plus de calcium est présent que de sodium sur une base d'équivalents molaires, et (a) le catalyseur comprend au moins 200 ppm en poids de sodium et/ou (b) le catalyseur comprend 1000 ppm en poids de calcium ;
ii. le promoteur contient du calcium et du potassium, plus de calcium est présent que de potassium sur une base d'équivalents molaires, et (a) le catalyseur comprend au moins 200 ppm en poids de potassium et/ou (b) le catalyseur comprend 1000 ppm en poids de calcium ; et
iii. le catalyseur contient du calcium et du magnésium, et le catalyseur comprend au moins 1000 ppm en poids de calcium.

2. Procédé selon la revendication 1, dans lequel le dinitrile est l'adiponitrile et dans lequel le procédé comprend en outre le contrôle de la concentration de promoteur pour réduire la sélectivité en HMI (hexaméthylèneimine) et BHMT (bis(hexaméthylène)triamine).

3. Procédé selon la revendication 2, comprenant en outre le contrôle de la concentration de promoteur en-dessous d'un niveau amenant le procédé à produire plus de AMCPA (aminométhylcyclopentamine) par unité de courant d'alimentation d'adiponitrile que l'opération du procédé dans des conditions équivalentes mais en l'absence du promoteur.

4. Procédé selon l'une quelconque des revendications précédentes, dans lequel le promoteur comprend au moins deux de sodium, calcium et magnésium.

5. Procédé selon la revendication 4, dans lequel le ratio molaire de Ca:Na dans le catalyseur est au moins 5:2.

6. Procédé selon l'une quelconque des revendications 1, 2 ou 3, dans lequel le promoteur contient du calcium et du potassium et dans lequel le ratio molaire de Ca:K est au moins 5:2.

7. Procédé selon l'une quelconque des revendications précédentes, dans lequel le promoteur est au moins l'un composé sélectionné dans le groupe constitué d'oxydes, d'hydroxydes et de carbonates.

8. Procédé selon l'une quelconque des revendications précédentes, dans lequel le promoteur est ajouté au catalyseur avant la mise en contact du catalyseur avec du dinitrile et de l'hydrogène.

9. Procédé selon l'une quelconque des revendications précédentes, dans lequel le dinitrile est mis en contact avec de l'hydrogène dans un réacteur et dans lequel le promoteur est présent dans le réacteur à une concentration comprise entre 500 ppm en poids et 3500 ppm en poids sur la base du poids du catalyseur.

10. Procédé selon l'une quelconque des revendications précédentes, dans lequel le promoteur comprend Ca(OH)₂.

11. Procédé selon l'une quelconque des revendications précédentes, dans lequel le promoteur comprend NaOH.

12. Procédé selon l'une quelconque des revendications précédentes, dans lequel le catalyseur comprend entre 200 ppm en poids et 800 ppm en poids de sodium.

13. Procédé selon l'une quelconque des revendications précédentes, dans lequel le catalyseur comprend entre 100 ppm en poids et 500 ppm en poids de potassium sur la base du poids du catalyseur.

14. Procédé selon l'une quelconque des revendications précédentes, dans lequel le dinitrile est l'adiponitrile et dans lequel le procédé comprend en outre la diminution de la formation de la somme de HMI et BHMT par au moins 5 % en poids, sur la base du poids de la somme de HMI et BHMT produit par substantiellement le même catalyseur dans des conditions de procédé substantiellement identiques excepté pour l'absence de promoteur.

15. Procédé selon l'une quelconque des revendications précédentes, dans lequel le dinitrile est l'adiponitrile et dans lequel le procédé comprend en outre la sélection d'une concentration de promoteur telle que la sélectivité en AMCPA soit augmentée de moins de 5 % en poids sur la base du poids d'AMCPA produite par substantiellement le même catalyseur dans des conditions de procédé substantiellement identiques excepté pour l'absence de promoteur.
